(19) Europäisches Patentamt
European Patent Office
Office européen des brevets

(11) EP 4 209 536 A1

(12) **EUROPEAN PATENT APPLICATION**
published in accordance with Art. 153(4) EPC

(43) Date of publication:
**12.07.2023 Bulletin 2023/28**

(21) Application number: **22867592.2**

(22) Date of filing: **19.08.2022**

(51) International Patent Classification (IPC):
***C08J 3/24*** *(2006.01)* ***C08F 220/34*** *(2006.01)*
***C08F 220/04*** *(2006.01)* ***A61L 15/60*** *(2006.01)*

(52) Cooperative Patent Classification (CPC):
**A61L 15/60; C08F 220/04; C08F 220/34; C08J 3/24**

(86) International application number:
**PCT/KR2022/012466**

(87) International publication number:
**WO 2023/038323 (16.03.2023 Gazette 2023/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.09.2021 KR 20210120837**
**18.08.2022 KR 20220103399**

(71) Applicant: **Lg Chem, Ltd.**
**Seoul 07336 (KR)**

(72) Inventors:
- **MOON, Hyeran**
  **Daejeon 34122 (KR)**
- **YUN, Haesung**
  **Daejeon 34122 (KR)**
- **CHOI, Hyungsam**
  **Daejeon 34122 (KR)**
- **JUNG, Seonjung**
  **Daejeon 34122 (KR)**
- **LEE, Ji Seok**
  **Daejeon 34122 (KR)**
- **HUR, Yoon Hyung**
  **Daejeon 34122 (KR)**
- **LEE, Hwanhee**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) SUPER ABSORBENT POLYMER AND PREPARATION METHOD THEREFOR

(57) Provided are a superabsorbent polymer and a method of preparing the same, more particularly, a superabsorbent polymer capable of exhibiting an improved bacterial growth-inhibitory property without deterioration in a water retention capacity of the superabsorbent polymer, and a method of preparing the same.

EP 4 209 536 A1

## Description

[Technical Field]

Cross-reference to Related Application

The present application is based on, and claims priority from, Korean Patent Application Nos. 10-2021-0120837, and 10-2022-0103399, filed on September 10, 2021, and August 18, 2022, respectively, the disclosures of which are hereby incorporated by reference herein in their entirety.

The present invention relates to a superabsorbent polymer exhibiting an improved bacterial growth-inhibitory property without deterioration in absorption performances of the superabsorbent polymer, and a method of preparing the same.

[Background Art]

A superabsorbent polymer (SAP) is a synthetic polymeric material capable of absorbing moisture from 500 to 1000 times its own weight. Various manufacturers have denominated it as different names, such as SAM (Super Absorbency Material), AGM (Absorbent Gel Material), etc. Since such superabsorbent polymers started to be practically applied in sanitary products, now they have been widely used not only for hygiene products such as paper diapers for children, etc., but also for water retaining soil products for gardening, water stop materials for the civil engineering and construction, sheets for raising seedling, fresh-keeping agents for food distribution fields, materials for poultice or the like.

In particular, superabsorbent polymers are most widely applied to hygiene products such as paper diapers for children, diapers for adults, or disposable absorbent products. Therefore, when bacteria grow in these hygiene products and disposable absorbent products, there is a problem in that secondary odors as well as various diseases are induced. Accordingly, attempts have been made to introduce various bacterial growth inhibitory components, or deodorizing or antibacterial functional components into superabsorbent polymers.

However, in the attempts to introduce antibacterial agents capable of inhibiting bacterial growth into superabsorbent polymers, it is not easy to select and introduce an antibacterial agent component that exhibits excellent bacterial growth-inhibiting and deodorizing properties, is harmless to the human body, and satisfies economic feasibility without deteriorating basic physical properties of the superabsorbent polymers.

Accordingly, there is a continuous demand for the development of a superabsorbent polymer-related technology capable of remarkably inhibiting bacterial growth without deteriorating basic physical properties of superabsorbent polymers.

[Disclosure]

[Technical Problem]

Accordingly, there are provided a superabsorbent polymer capable of exhibiting an improved bacterial growth-inhibiting property without deterioration in absorption performances of the superabsorbent polymer, and a method of preparing the same.

[Technical Solution]

According to one embodiment of the present invention, there is provided a superabsorbent polymer including a base polymer including a crosslinked polymer which is obtained by polymerizing an acrylic acid-based monomer including acidic groups of which at least part is neutralized, a polymerizable antibacterial monomer represented by the following Chemical Formula 1, and a first crosslinking agent,

wherein at least part of the base polymer is surface-treated by a second crosslinking agent:

[Chemical Formula 1]

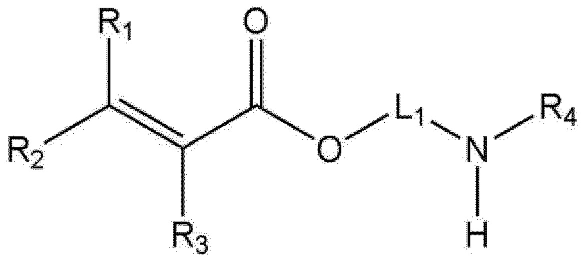

in Chemical Formula 1,
$R_1$ to $R_3$ are each independently hydrogen or methyl,
$R_4$ is hydrogen or a substituted or unsubstituted alkyl having 1 to 20 carbon atoms, and
$L_1$ is a substituted or unsubstituted alkylene having 1 to 10 carbon atoms.

According to another embodiment of the present invention, there is provided a method of preparing a superabsorbent polymer, the method including the steps of:

forming a water-containing gel polymer by performing a crosslinking polymerization of an acrylic acid-based monomer including acidic groups of which at least part is neutralized, and a polymerizable antibacterial monomer represented by the following Chemical Formula 1 in the presence of a first crosslinking agent and a polymerization initiator;
forming a base polymer including a crosslinked polymer by drying, pulverizing, and classifying the water-containing gel polymer; and
surface-crosslinking the base polymer by heat treatment in the presence of a second crosslinking agent:

[Chemical Formula 1]

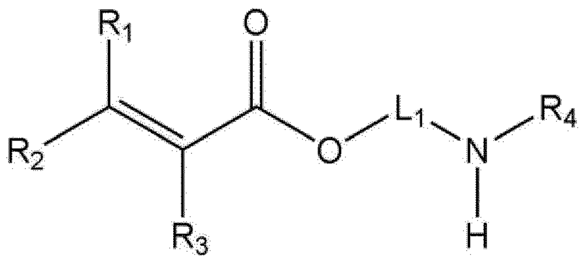

in Chemical Formula 1,
$R_1$ to $R_3$ are each independently hydrogen or methyl,
$R_4$ is hydrogen or a substituted or unsubstituted alkyl having 1 to 20 carbon atoms, and
$L_1$ is a substituted or unsubstituted alkylene having 1 to 10 carbon atoms.

Furthermore, according to still another embodiment of the present invention, there is provided a hygiene product including the above-described superabsorbent polymer.

[Effect of the Invention]

A superabsorbent polymer of the present invention may exhibit an antibacterial property of inhibiting bacterial growth which may be harmful to the human body and may cause secondary odors.
Specifically, since the superabsorbent polymer is prepared by using a polymerizable antibacterial monomer having a specific structure during formation of a crosslinked polymer, it may exhibit an antibacterial property against at least one of Gram-negative bacteria while maintaining excellent water retention capacity, unlike those prepared by using another antibacterial agent, and the used antibacterial monomer does not remain in the polymer, which does not cause a safety problem in the human body due to leakage of the antibacterial agent.
Accordingly, the superabsorbent polymer may be very preferably applied to various hygiene products, such as diapers for children as well as diapers for adults requiring an antibacterial property against bacteria, etc.

[Best Mode for Carrying Out the Invention]

The terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components or combinations thereof beforehand.

Further, in the present invention, when a layer or an element is mentioned to be formed "on" or "above" layers or elements, it means that each layer or element is directly formed on the layers or elements, or other layers or elements may be formed between the layers, subjects, or substrates.

The present invention may be variously modified and have various forms, and specific exemplary embodiments are exemplified and explained in detail in the following description. However, it is not intended to limit the present invention to the specific exemplary embodiments and it must be understood that the present invention includes every modifications, equivalents, or replacements included in the spirit and technical scope of the present invention.

Further, the technical terms used in this description are just for mentioning specific exemplary embodiments and it is not intended to restrict the present invention. The singular expression used herein may include the plural expression unless it is differently expressed contextually.

Meanwhile, as used herein, the term "(meth)acrylate" includes both acrylates and methacrylates.

Further, as used herein, the alkyl group may be linear or branched, and its number of carbon atoms is, but not particularly limited to, preferably 1 to 20. According to one embodiment, the number of carbon atoms of the alkyl group is 1 to 10. According to another embodiment, the number of carbon atoms of the alkyl group is 1 to 6. Specific examples of the alkyl group may include methyl, ethyl, propyl, n-propyl, isopropyl, butyl, n-butyl, isobutyl, tert-butyl, sec-butyl, 1-methyl-butyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, etc., but are not limited thereto. In the present specification, the description of the above-described alkyl group may be applied to alkylene, except that alkylene is a divalent group.

As used herein, the term "polymer" refers to a polymerized state of acrylic acid-based monomers, and may encompass those of all water content ranges or particle size ranges. Among the polymers, those having a water content (a moisture content) of about 40% by weight or more after being polymerized and before being dried may be referred to as a water-containing gel polymer, and particles obtained by pulverizing and drying the water-containing gel polymer may be referred to as a crosslinked polymer.

Further, the term "superabsorbent polymer particle" refers to a particulate material including a crosslinked polymer which is obtained by polymerizing and crosslinking the acrylic acid-based monomer including acidic groups of which at least part is neutralized, by a first crosslinking agent.

Further, the term "superabsorbent polymer" refers to, depending on the context, a crosslinked polymer obtained by polymerizing the acrylic acid-based monomer including acidic groups of which at least part is neutralized, or a base polymer in the form of powder, consisting of superabsorbent polymer particles obtained by pulverizing the crosslinked polymer, or is used to encompass those made suitable for commercialization through additional processes of the crosslinked polymer or the base polymer, for example, surface crosslinking, reassembling of fine particles, drying, pulverizing, classifying, etc.

Traditionally, in order to secure antibacterial and deodorizing properties in the superabsorbent polymers, a metal compound having an antibacterial function or an organic compound containing a cation or alcohol functional group was introduced in the form of an additive. In this case, however, safety of the superabsorbent polymer was lowered, basic physical properties, such as absorption properties, etc., were lowered, and there were problems in persistence of antibacterial properties and leakage of antibacterial substances.

For example, an attempt has been made to introduce, into superabsorbent polymers, an antibacterial agent component containing an antibacterial metal ion such as silver, copper, copper, zinc, etc. This antibacterial metal ion-containing component may impart deodorizing properties by destroying the cell walls of microorganisms, such as bacteria, etc., and by killing bacteria with enzymes that may cause bad odor in the superabsorbent polymers. However, the metal ion-containing component is classified as a biocide material that may kill even microorganisms beneficial to the human body. As a result, when the superabsorbent polymers are applied to hygiene products such as diapers for children or adults, etc., introduction of the metal ion-containing antibacterial agent component is excluded as much as possible.

Traditionally, when the antibacterial agent inhibiting bacterial growth was introduced into a superabsorbent polymer, a method of mixing the superabsorbent polymer with a small amount of the antibacterial agent was mainly applied. However, when this mixing method is applied, it was difficult to uniformly maintain the bacterial growth-inhibiting

property over time. Moreover, this mixing method may cause nonuniform coating property and detachment of the antibacterial agent component during the process of mixing the superabsorbent polymer and the antibacterial agent, and there have been also disadvantages such as a need to install a new facility for the mixing.

Further, there are various types of bacteria such that more than 5,000 types of bacteria have been identified. Specifically, bacteria have a variety of cell morphologies such as a sphere shape, a rod shape, a spiral shape, etc., and the oxygen demand is also different between bacteria, and thus bacteria are divided into aerobic bacteria, facultative aerobic bacteria, and anaerobic bacteria. Therefore, it has not been easy for one type of antibacterial agent to have a physical/chemical mechanism by which cell membranes/cell walls of many different bacteria are damaged or proteins thereof are denatured.

However, it was found that when a superabsorbent polymer is prepared by polymerizing an acrylic acid-based monomer with a monomer containing a secondary amine of a specific structure, it exhibits absorption performances above a predetermined level while exhibiting an antibacterial property against at least one of Gram-negative bacteria, thereby completing the present invention. When the amino group of a polymerizable antibacterial monomer represented by Chemical Formula 1 binds with a proton to be positively charged, the interaction with the cell wall of microorganisms increases, and the alkyl group corresponding to the pendant group destabilizes the cell wall of the microorganisms and inhibits the growth of the microorganisms, thereby exhibiting the antibacterial property. Since most bacterial cell membranes are negatively charged, most antibacterial substances have a positive charge. When a hydrogen cation (proton) binds to the amine of the compound represented by Chemical Formula 1 to exhibit a cationic property, it may interact with the cell membrane of bacteria. Thus, the compound represented by Chemical Formula 1 has an antibacterial property.

Moreover, since the superabsorbent polymer includes the antibacterial monomer in the form of the crosslinked polymer, in which the antibacterial monomer is crosslinked with the acrylic acid-based monomer, the antibacterial monomer does not remain in the form of a compound in the superabsorbent polymer. Accordingly, there is no concern about leakage of the antibacterial agent even over time, and thus the superabsorbent polymer is characterized by exhibiting excellent stability.

Hereinafter, a superabsorbent polymer and a preparation method thereof will be described in more detail according to specific embodiments of the present invention.

**Superabsorbent polymer**

Specifically, a superabsorbent polymer according to one embodiment of the present invention is characterized by including a base polymer including a crosslinked polymer which is obtained by polymerizing an acrylic acid-based monomer including acidic groups of which at least part is neutralized, a polymerizable antibacterial monomer represented by the following Chemical Formula 1, and a first crosslinking agent, wherein at least part of the base polymer is surface-treated by a second crosslinking agent:

[Chemical Formula 1]

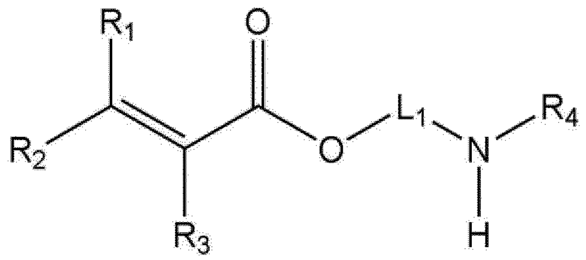

in Chemical Formula 1,

$R_1$ to $R_3$ are each independently hydrogen or methyl,

$R_4$ is hydrogen or a substituted or unsubstituted alkyl having 1 to 20 carbon atoms, and

$L_1$ is a substituted or unsubstituted alkylene having 1 to 10 carbon atoms.

In this regard, the crosslinked polymer, resulting from crosslinking polymerization of the acrylic acid-based monomer and the polymerizable antibacterial monomer in the presence of the first crosslinking agent, has a three-dimensional network structure, in which the main chains formed by polymerization of the monomers are crosslinked by the first crosslinking agent. Therefore, the polymerizable antibacterial monomer does not exist as a separate compound in the superabsorbent polymer, but exists as a repeating unit constituting the main chain, and thus it does not leak over time. Accordingly, the antibacterial property of the superabsorbent polymer may be continuously maintained.

In Chemical Formula 1, preferably, $R_1$ and $R_2$ are each hydrogen, and $R_3$ may be methyl.

Further, preferably, $R_4$ may be a substituted or unsubstituted alkyl having 1 to 10 carbon atoms, and more preferably, $R_4$ may be tert-butyl. In general, as the number of carbon atoms at the $R_4$ position is larger, affinity with the double layer of the outer cell wall of microorganisms is higher, thereby exhibiting strong antibacterial activity. In the case of alkyl having 8 or more carbon atoms, it is important to have an appropriate length because of exhibiting a strong bactericidal property even in water.

When the end of the antibacterial compound is a tertiary amine, it may play a role in interacting with the outer cell wall of microorganisms as described above. However, the electron inductive effect does not increase, but it negatively affects the interaction between the cell membrane and the positive charge due to the steric effect. In contrast, the end of the compound represented by Chemical Formula 1 is a secondary amine, and in this case, the steric effect becomes smaller and it more easily binds with protons to become positively charged, thereby exhibiting a stronger interaction with the negatively charged cell wall of microorganisms. Therefore, even when $R_4$ is tertbutyl having a relatively small number of carbon atoms, excellent antibacterial performance may be exhibited.

Preferably, $L_1$ may be a substituted or unsubstituted alkylene having 1 to 5 carbon atoms, and more preferably, $L_1$ may be ethylene.

For example, the polymerizable antibacterial monomer may be a compound represented by the following Chemical Formula 1-1:

[Chemical Formula 1-1]

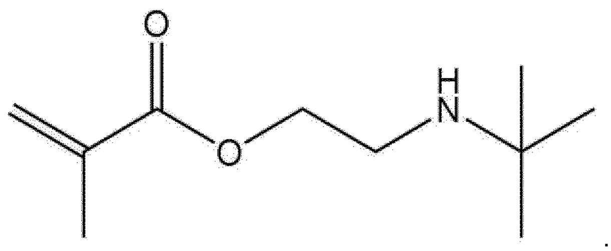

Meanwhile, the polymerizable antibacterial monomer is included in the crosslinked polymer in an amount of 5 parts by weight or more and 20 parts by weight or less with respect to 100 parts by weight of the acrylic acid-based monomer. When the polymerizable antibacterial monomer is included in an amount of less than 5 parts by weight with respect to 100 parts by weight of the acrylic acid-based monomer, it is difficult to exhibit the sufficient antibacterial effect. When the polymerizable antibacterial monomer is included in an amount of more than 20 parts by weight with respect to 100 parts by weight of the acrylic acid-based monomer, the absorption performances of the superabsorbent polymer may deteriorate, and the polymerization rate of the superabsorbent polymer may decrease.

More specifically, the polymerizable antibacterial monomer is included in the crosslinked polymer in an amount of 5 parts by weight or more, 5.5 parts by weight or more, or 6 parts by weight or more, and 20 parts by weight or less, 15 parts by weight or less, 12 parts by weight or less, 10 parts by weight or less, 9 parts by weight or less, or 8 parts by weight or less with respect to 100 parts by weight of the acrylic acid-based monomer. At this time, in terms of maintaining absorption performances, specifically, centrifuge retention capacity of the superabsorbent polymer, the polymerizable antibacterial monomer is included in the crosslinked polymer in an amount of 5 parts by weight to 10 parts by weight with respect to 100 parts by weight of the acrylic acid-based monomer.

In this regard, the polymerizable antibacterial monomer is included in the crosslinked polymer in an amount of 5 parts by weight to 20 parts by weight with respect to 100 parts by weight of the acrylic acid-based monomer, which means that the polymerizable antibacterial monomer is used in an amount of 5 parts by weight to 20 parts by weight with respect to 100 parts by weight of the acrylic acid-based monomer during the preparation of the crosslinked polymer. This may be confirmed by whether or not the antibacterial monomer is detected when examining the residual monomer of the superabsorbent polymer. In the superabsorbent polymer, the antibacterial monomer was not detected after preparation, which may indicate that all of the used antibacterial monomer was consumed in the polymerization with the acrylic acid-based monomer.

For example, the polymerizable antibacterial monomer represented by Chemical Formula 1-1 exhibits the antibacterial property against specific bacteria. Here, the "exhibiting the antibacterial property against specific bacteria" means that the number of bacteria cultured after allowing a material to be tested for the antibacterial property to absorb artificial urine inoculated with test bacteria is remarkably reduced, as compared to the number of reference bacteria cultured after allowing a material without the antibacterial material to absorb artificial urine inoculated with test bacteria. Specifically, as the bacterial growth-inhibitory rate calculated by the following Equation 1 according to the antibacterial

property test to be described later is higher, the antibacterial property is better.

[Equation 1]

$$\text{Bacterial growth-inhibitory rate(\%)} = [(OD_{Reference} - OD_{Sample})/OD_{Reference}] * 100$$

in Equation, $OD_{Reference}$ represents absorbance of a culture medium containing no antibacterial material, and $OD_{sample}$ represents absorbance of a culture medium containing the antibacterial material.

Alternatively, the "exhibiting the antibacterial property against specific bacteria" means that a bacteriostatic reduction rate (%) calculated by the following Equation 2 is 45% or more.

[Equation 2]

$$\text{Bacteriostatic reduction rate (\%)} = (1 - C_{sample} / C_{Reference}) * 100$$

in Equation, $C_{sample}$ represents a concentration (Co) of microorganisms in a culture medium of a material containing the antibacterial material, and $C_{Reference}$ represents a concentration (Co) of microorganisms in a culture medium of a material containing no antibacterial material.

More preferably, the "exhibiting the antibacterial property against specific bacteria" means that the bacteriostatic reduction rate (%) calculated by Equation 2 is 60% or more, 70% or more, 80% or more, 90% or more, 95% or more, or 99% or more.

Specifically, the polymerizable antibacterial monomer represented by Chemical Formula 1-1 exhibits the antibacterial property against *Escherichia coli (E. coli)* or *Proteus mirabilis (P. mirabilis).* The polymerizable antibacterial monomer represented by Chemical Formula 1-1 exhibits the antibacterial property against *Escherichia coli (E. coli)* or *Proteus mirabilis (P. mirabilis),* even when used in an amount of 3 parts by weight or more and 7 parts by weight less with respect to 100 parts by weight of the superabsorbent polymer.

Meanwhile, the acrylic acid-based monomer is a compound represented by the following Chemical Formula 1:

[Chemical Formula 2] R-COOM'

in Chemical Formula 2,
R is an alkyl group containing an unsaturated bond and having 2 to 5 carbon atoms, and
M' is a hydrogen atom, a monovalent or divalent metal, an ammonium group, or an organic amine salt.

Preferably, the monomer may include one or more selected from the group consisting of (meth)acrylic acid, and a monovalent (alkali) metal salt thereof, a divalent metal salt thereof, an ammonium salt thereof, and an organic amine salt thereof.

As described, when (meth)acrylic acid and/or a salt thereof may be used as the acrylic acid-based monomer, it is advantageous in that a superabsorbent polymer having improved absorbency may be obtained.

Here, the acrylic acid-based monomer may have acidic groups of which at least part is neutralized. Preferably, those partially neutralized with an alkali material such as sodium hydroxide, potassium hydroxide, ammonium hydroxide, etc. may be used as the monomer. In this regard, a degree of neutralization of the acrylic acid-based monomer may be 40 mol% to 95 mol%, or 40 mol% to 80 mol%, or 45 mol% to 75 mol%. The range of the degree of neutralization may vary depending on final physical properties. However, an excessively high degree of neutralization renders the neutralized monomers precipitated, and thus polymerization may not occur readily, whereas an excessively low degree of neutralization not only greatly deteriorates absorbency of the polymer but also endows the polymer with hard-to-handle properties, such as of elastic rubber.

Further, as used herein, the term 'first crosslinking agent' is used to distinguish it from a second crosslinking agent which crosslinks the surface of the superabsorbent polymer particle, described later, and functions to polymerize the acrylic acid-based monomers and polymerizable antibacterial monomers by crosslinking unsaturated bonds thereof. The crosslinking in the above step is performed regardless of surface or internal crosslinking. However, when the surface crosslinking process of the superabsorbent polymer particles to be described later is performed, the particle surface of the superabsorbent polymer finally prepared has a crosslinked structure by the second crosslinking agent, and the inside thereof has a crosslinked structure by the first crosslinking agent. Therefore, since the second crosslinking agent mainly serves to crosslink the surface of the superabsorbent polymer, it may serve as a surface crosslinking agent, and the

first crosslinking agent is distinguished from the second crosslinking agent, and may serve as an internal crosslinking agent.

As the first crosslinking agent, any compound is possible as long as it enables introduction of crosslinkage during polymerization of the acrylic acid-based monomer. Non-limiting examples of the first crosslinking agent may include multifunctional crosslinking agents, such as N,N'-methylenebisacrylamide, trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, or ethylene carbonate, which may be used alone or in combination of two or more thereof, but are not limited thereto.

Preferably, as the first crosslinking agent, polyalkylene glycol di(meth)acrylate-based compounds, such as polyethylene glycol (meth)acrylate, polyethylene glycol di(meth)acrylate, propylene glycol di(meth)acrylate, or polypropylene glycol (meth)acrylate may be used.

The crosslinking polymerization of the acrylic acid-based monomer in the presence of the first crosslinking agent may be performed by thermal polymerization, photopolymerization, or hybrid polymerization in the presence of a polymerization initiator, as needed, a thickener, a plasticizer, a preservation stabilizer, an antioxidant, etc., and a detailed description thereof will be described later.

Meanwhile, the superabsorbent polymer includes a surface-crosslinked layer formed on the crosslinked polymer by additionally crosslinking the base polymer including the crosslinked polymer via the second crosslinking agent. The surface-crosslinked layer includes surface-treatment of at least part of the base polymer. This is to increase the surface crosslinking density of the superabsorbent polymer particles. As described, when the superabsorbent polymer particle further includes the surface-crosslinked layer, it may have a structure in which the external crosslinking density is higher than the internal crosslinking density.

As the second crosslinking agent, second crosslinking agents which have been used in the preparation of the superabsorbent polymer may be used without particular limitation. For example, the second crosslinking agent may include one or more polyols selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol, and glycerol; one or more carbonate-based compounds selected from the group consisting of ethylene carbonate, propylene carbonate, and glycerol carbonate; epoxy compounds such as ethylene glycol diglycidyl ether, etc.; oxazoline compounds such as oxazolidinone, etc.; polyamine compounds; oxazoline compounds; mono-, di-, or polyoxazolidinone compounds; metal-containing sulfates such as aluminum sulfate, etc., or one or more polyvalent metal salts of carboxylates; or cyclic urea compounds, etc.

Specifically, as the second crosslinking agent, one or more, two or more, or three or more of the above-described second crosslinking agents may be used. For example, ethylene carbonate, propylene glycol, and/or aluminum sulfate may be used.

Further, the superabsorbent polymer may be in the form of particles having a particle size of 850 μm or less, for example, about 150 μm to about 850 μm. In this regard, the particle size may be measured in accordance with European Disposables and Nonwovens Association (EDANA) standard WSP 220.3 method. Here, when the superabsorbent polymer includes a large amount of fine particles having a particle size of less than 150 μm, general physical properties of the superabsorbent polymer may be deteriorated, which is not preferred.

Meanwhile, the superabsorbent polymer may exhibit the antibacterial property against at least one of Gram-negative bacteria, as described above. More specifically, the superabsorbent polymer may exhibit the antibacterial property against one or more bacteria classified as Gram-negative bacteria.

The Gram-negative bacteria collectively refer to bacteria that are stained red when stained by the Gram staining method. The Gram-negative bacteria have outer membrane consisting of lipopolysaccharides, lipoproteins, and other complex polymeric materials, instead of the cell wall having a relatively small amount of peptidoglycan, as compared with the Gram-positive bacteria. Therefore, when the Gram-negative bacteria are stained with a basic dye such as crystal violet and then treated with ethanol, they are decolorized, and when counter-stained with a red dye such as safranin, they are colored red. Bacteria classified as the Gram-negative bacteria include *Proteus Mirabilis, Escherichia coli, Salmonella typhi, Pseudomonas aeruginosa,* or *Vibrio cholerae,* etc.

Since the Gram-negative bacteria may cause various diseases upon contact, and may also cause secondary infection in severe patients with weakened immune systems, it is preferable that a single antibacterial agent is used to achieve the antibacterial property against one or more of the Gram-negative bacteria. In this regard, the Gram-negative bacteria against which the superabsorbent polymer exhibits the antibacterial property may be *Escherichia coli* or *Proteus Mirabilis,* but is not limited thereto.

Further, the superabsorbent polymer may exhibit a 30-min centrifuge retention capacity (CRC) of 20 g/g or more and 45 g/g or less for physiological saline (0.9 wt% aqueous sodium chloride solution), as measured according to

the EDANA method WSP 241.3. When the centrifuge retention capacity (CRC) is less than 20 g/g, the ability to retain a liquid after absorption is reduced, and thus the superabsorbent polymer is not suitable for application to hygiene products. More specifically, the centrifuge retention capacity (CRC) of the superabsorbent polymer may be 20 g/g or more, 25 g/g or more, 30 g/g or more, 35 g/g or more, or 35.6 g/g or more, and 45 g/g or less, 43 g/g or less, 41 g/g or less, 40 g/g or less, 38 g/g or less, or 37.4 g/g or less.

Accordingly, the above-described superabsorbent polymer including a predetermined amount of the polymerizable antibacterial monomer in the crosslinked polymer may have a centrifuge retention capacity (CRC) of 20 g/g to 45 g/g, and at the same time, may exhibit the excellent antibacterial property.

## Method of preparing superabsorbent polymer

Meanwhile, the superabsorbent polymer may be prepared by the following preparation method including the steps of:

forming a water-containing gel polymer by performing a crosslinking polymerization of an acrylic acid-based monomer including acidic groups of which at least part is neutralized, and a polymerizable antibacterial monomer represented by Chemical Formula 1 in the presence of a first crosslinking agent and a polymerization initiator;

forming a base polymer including a crosslinked polymer by drying, pulverizing, and classifying the water-containing gel polymer; and

surface-crosslinking the base polymer by heat treatment in the presence of a second crosslinking agent.

First, the step 1 is a step of forming a water-containing gel polymer by performing a crosslinking polymerization of an acrylic acid-based monomer including acidic groups of which at least part is neutralized, and a polymerizable antibacterial monomer in the presence of a first crosslinking agent and a polymerization initiator.

The step may consist of a step of preparing a monomer composition by mixing the acrylic acid-based monomer, the first crosslinking agent, and the polymerization initiator, and a step of forming the water-containing gel polymer by performing thermal polymerization or photopolymerization of the monomer composition. In this regard, for descriptions of the acrylic acid-based monomer, the polymerizable antibacterial monomer, and the first crosslinking agent, reference may be made to those described above.

In the monomer composition, the first crosslinking agent is included in an amount of 0.01 part by weight to 1 part by weight with respect to 100 parts by weight of the acrylic acid-based monomer, thereby crosslinking the polymerized polymer. When the amount of the first crosslinking agent is less than 0.01 part by weight, the improvement effect due to crosslinking is insignificant. When the amount of the first crosslinking agent is more than 1 part by weight, absorbency of the superabsorbent polymer may be reduced. More specifically, the first crosslinking agent may be included in an amount of 0.05 parts by weight or more, or 0.1 part by weight or more, and 0.5 parts by weight or less, or 0.3 parts by weight or less with respect to 100 parts by weight of the acrylic acid-based monomer.

Further, the polymerization initiator may be appropriately selected depending on a polymerization method. When a thermal polymerization method is used, a thermal polymerization initiator is used. When a photopolymerization method is used, a photopolymerization initiator is used. When a hybrid polymerization method (a method of using both heat and light) is used, both the thermal polymerization initiator and the photopolymerization initiator are used. However, even though the photopolymerization method is used, a certain amount of heat is generated by light irradiation such as UV irradiation, etc., and is also generated with the polymerization reaction which is an exothermic reaction. Therefore, the thermal polymerization initiator may be further used.

As the photopolymerization initiator, any compound capable of forming radicals by a light such as UV may be used without limitations in view of its composition.

For example, one or more selected from the group consisting of benzoin ether, dialkyl acetophenone, hydroxyl alkylketone, phenyl glyoxylate, benzyl dimethyl ketal, acyl phosphine, and $\alpha$-aminoketone may be used as the photopolymerization initiator. Meanwhile, specific examples of acyl phosphine may include diphenyl(2,4,6-trimethylbenzoyl)phosphine oxide, phenylbis(2,4,6-trimethylbenzoyl)phosphine oxide, ethyl (2,4,6-trimethylbenzoyl) phenylphosphinate, etc. More various photopolymerization initiators are well disclosed in "UV Coatings: Basics, Recent Developments and New Application (Elsevier, 2007)" written by Reinhold Schwalm, p115, however, they are not limited to the above described examples.

The photopolymerization initiator may be included in an amount of 0.001 part by weight to 1 part by weight with respect to 100 parts by weight of the acrylic acid-based monomer. When the amount of the photopolymerization initiator is less than 0.001 part by weight, the polymerization rate may become slow, and when the amount of the photopolymerization initiator is more than 1 part by weight, a molecular weight of the superabsorbent polymer becomes small and its physical properties may become uneven. More specifically, the photopolymerization initiator may be included in an amount of 0.005 parts by weight or more, or 0.01 part by weight or more, or 0.1 part by weight or more, and 0.5 parts

by weight or less, or 0.3 parts by weight or less with respect to 100 parts by weight of the acrylic acid-based monomer.

Further, when the thermal polymerization initiator is further included as the polymerization initiator, one or more selected from the group consisting of a persulfate-based initiator, an azo-based initiator, hydrogen peroxide, and ascorbic acid may be used as the thermal polymerization initiator. Specific examples of the persulfate-based initiator may include sodium persulfate ($Na_2S_2O_8$), potassium persulfate ($K_2S_2O_8$), ammonium persulfate ($(NH_4)_2S_2O_8$), etc., and examples of the azo-based initiator may include 2,2-azobis-(2-amidinopropane)dihydrochloride, 2,2-azobis-(N,N-dimethylene)isobutyramidine dihydrochloride, 2-(carbamoylazo)isobutyronitrile, 2,2-azobis[2-(2-imidazolin-2-yl)propane]dihydrochloride, 4,4-azobis-(4-cyanovaleric acid), etc. More various thermal polymerization initiators are well disclosed in 'Principle of Polymerization(Wiley, 1981)', written by Odian, p 203, however, the thermal polymerization initiator is not limited to the above-described examples.

The thermal polymerization initiator may be included in an amount of 0.001 part by weight to 1 part by weight with respect to 100 parts by weight of the acrylic acid-based monomer. When the amount of the thermal polymerization initiator is less than 0.001 part by weight, additional thermal polymerization hardly occurs, and thus the addition effect of the thermal polymerization initiator may be insignificant. When the amount of the thermal polymerization initiator is more than 1 part by weight, the molecular weight of the superabsorbent polymer may become low and its physical properties may become uneven. More specifically, the thermal polymerization initiator may be included in an amount of 0.005 parts by weight or more, or 0.01 part by weight or more, or 0.1 part by weight or more, and 0.5 parts by weight or less, or 0.3 parts by weight or less with respect to 100 parts by weight of the acrylic acid-based monomer.

In addition to the polymerization initiators, one or more kinds of additives such as a surfactant, a thickener, a plasticizer, a preservation stabilizer, an antioxidant, etc., may be further included, as needed, during crosslinking polymerization.

The above-described monomer composition including the acrylic acid-based monomer, the polymerizable antibacterial monomer, and the first crosslinking agent, and optionally, the photopolymerization initiator, and the additives may be in the form of being dissolved in a solvent.

As the solvent to be applicable, any solvent may be used without limitations in view of composition as long as it is able to dissolve the above components, and for example, one or more selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, and N,N-dimethylacetamide may be used in combination. The solvent may be included in the remaining amount excluding the above-described components, with respect to the total amount of the monomer composition.

Further, when a water-soluble solvent such as water is used as the solvent and a terpene-based compound having no solubility for water is used as the polymerizable antibacterial monomer, a surfactant may be additionally added in the amount of 10 parts by weight or less with respect to 100 parts by weight of the polymerizable antibacterial monomer in order to increase the solubility.

Meanwhile, the method of forming the water-containing gel polymer by thermal polymerization or photopolymerization of the monomer composition is also not particularly limited in view of constitution, as long as it is a polymerization method commonly used.

Specifically, the photopolymerization may be performed by irradiating UV with an intensity of 3 mW to 30 mW, or 10 mW to 20 mW at a temperature of 60°C to 90°C, or 70°C to 80°C. When the photopolymerization may be performed under these conditions, it is possible to form the crosslinked polymer with higher polymerization efficiency.

Further, when the photopolymerization is performed, it may be performed in a reactor equipped with a movable conveyor belt or in a stainless steel container with a predetermined size, but the above-described polymerization method is an example only, and the present invention is not limited to the above-described polymerization methods.

Further, as described above, when the photopolymerization is performed in a reactor equipped with a movable conveyor belt, the obtained water-containing gel polymer may be usually a sheet-like water-containing gel polymer having a width of the belt. In this case, a thickness of the polymer sheet may vary depending on the concentration of the monomer composition fed thereto and the feeding speed, and usually, it is preferable to supply the monomer composition such that a sheet-like polymer having a thickness of about 0.5 cm to about 5 cm may be obtained. When the monomer composition is supplied to such an extent that the thickness of the sheet-like polymer becomes too thin, it is undesirable because the production efficiency is low, and when the thickness of the sheet-like polymer is more than 5 cm, the polymerization reaction may not evenly occur over the entire thickness because of the excessive thickness.

Further, a water content of the water-containing gel polymer obtained by the above-mentioned method may be about 40% by weight to about 80% by weight with respect to the total weight of the water-containing gel polymer. Meanwhile, the "water content" as used herein means a weight occupied by water with respect to the total weight of the water-containing gel polymer, which may be a value obtained by subtracting the weight of the dried polymer from the weight of the water-containing gel polymer. Specifically, the water content may be defined as a value calculated by

measuring the weight loss due to evaporation of moisture in the polymer during a process of drying by raising the temperature of the polymer through infrared heating. At this time, the water content is measured under the following drying conditions: the temperature is increased from room temperature to about 180°C and then the temperature is maintained at 180°C, and the total drying time is set to 20 minutes, including 5 minutes for the temperature rising step.

Meanwhile, after the preparation of the water-containing gel polymer, a process of coarsely pulverizing the prepared water-containing gel polymer may be optionally performed, before subsequent drying and pulverizing processes.

The coarse pulverization process is a process for increasing the drying efficiency in the subsequent drying process and controlling the particle size of the prepared superabsorbent polymer powder. In this regard, a pulverizer used here is not limited to its configuration, and specifically, it may include any one selected from the group consisting of a vertical pulverizer, a turbo cutter, a turbo grinder, a rotary cutter mill, a cutter mill, a disc mill, a shred crusher, a crusher, a meat chopper, and a disc cutter, but is not limited to the above-described examples.

The coarse pulverization process may be performed, for example, such that the water-containing gel polymer may have a particle size of about 2 mm to about 10 mm. Pulverization of the water-containing gel polymer to a particle size of smaller than 2 mm is not technically easy due to the high water content of the water-containing gel polymer, and an agglomeration phenomenon between the pulverized particles may occur. Meanwhile, when the water-containing gel polymer is pulverized to a particle size of larger than 10 mm, the effect of increasing the efficiency in the subsequent drying step may be insignificant.

Next, the step 2 is a step of forming a base polymer including a crosslinked polymer by drying, pulverizing, and classifying the water-containing gel polymer prepared in the step 1.

The drying method may be selected and used without limitation in view of constitution, as long as it is commonly used in the process of drying the water-containing gel polymer. Specifically, the drying step may be performed by a method such as hot air supply, infrared irradiation, microwave irradiation, ultraviolet irradiation, etc.

Specifically, the drying may be performed at a temperature of about 120°C to about 250°C. When the drying temperature is lower than 120°C, the drying time becomes too long and the physical properties of the superabsorbent polymer finally formed may be deteriorated. When the drying temperature is higher than 250°C, only the polymer surface is excessively dried, and thus fine particles may be generated during the subsequent pulverization process and the physical properties of the superabsorbent polymer finally formed may be deteriorated. Therefore, the drying may be preferably performed at a temperature of 150°C or higher, or 160°C higher, and 200°C or lower, or 180°C or lower.

Meanwhile, the drying time may be about 20 min to about 90 min, in consideration of the process efficiency, but is not limited thereto.

The polymer after such a drying step may have a water content of about 5% by weight to about 10% by weight.

After the drying process, a pulverization process is performed. The pulverization process may be performed such that the polymer powder, i.e., the base polymer has a particle size of about 150 μm to about 850 μm. A pulverizer which is used for pulverization to such a particle size may include specifically a pin mill, a hammer mill, a screw mill, a roll mill, a disc mill, a jog mill, etc., but the present invention is not limited to the above-descried examples.

After the above pulverization step, to manage the physical properties of the superabsorbent polymer to be finally commercialized, the pulverized polymer powder may be further subjected to a classifying process according to the particle size.

The base polymer resulting from the above process may have a fine powder form including the crosslinked polymer obtained by crosslinking polymerizing the acrylic acid-based monomer and the polymerizable antibacterial monomer via the first crosslinking agent. Specifically, the superabsorbent polymer may have a fine powder form having a particle size of 150 μm to 850 μm or about 300 μm to about 600 μm.

Next, the step (step 3) of performing surface crosslinking of the base polymer prepared in the step 2 by heat treatment in the presence of a second crosslinking agent may be further included.

The surface crosslinking is a step of increasing the crosslinking density in the vicinity of the surface of the superabsorbent polymer in connection with the internal crosslinking density of particles. In general, the second crosslinking agent is applied to the surface of the polymer. Therefore, this reaction occurs on the surface of the polymer particle, which improves the crosslinking property on the surface of the particle without substantially affecting the interior of the particle. Thus, the surface-crosslinked superabsorbent polymer has a higher level of crosslinking in the vicinity of the surface than in the interior.

Such a second crosslinking agent may be used in an amount of about 0.001 part by weight to about 5 parts by weight with respect to 100 parts by weight of the water-containing gel polymer. For example, the second crosslinking agent may be used in an amount of about 0.005 parts by weight or more, 0.01 part by weight or more, or 0.05 parts by weight or more, and 5 parts by weight or less, 4 parts by weight or less, or 3 parts by weight or less with respect to 100 parts by weight of the superabsorbent polymer. It is possible to prepare a superabsorbent polymer exhibiting excellent general absorption properties by controlling the amount of the second crosslinking agent in the above-described range.

Further, a method of mixing the second crosslinking agent with the superabsorbent polymer is not limited in

view of its constitution. For example, a method of feeding the second crosslinking agent and the superabsorbent polymer to a reactor and mixing them with each other, a method of spraying the second crosslinking agent onto the superabsorbent polymer, or a method of mixing the superabsorbent polymer and the second crosslinking agent while continuously feeding them to a mixer which is continuously operated may be used.

In addition to the second crosslinking agent, water and alcohol are additionally mixed together, and may added in the form of a surface crosslinking solution. When water and alcohol are added, there is an advantage in that the second crosslinking agent may be uniformly dispersed in the superabsorbent polymer powder. Here, water and alcohol may be added in an amount of about 5 parts by weight to about 12 parts by weight with respect to 100 parts by weight of the polymer for the purpose of inducing uniform dispersion of the second crosslinking agent, preventing agglomeration of the superabsorbent polymer powder, and optimizing the surface penetration depth of the crosslinking agent at the same time.

The surface crosslinking reaction may be carried out by heating the superabsorbent polymer powder, to which the second crosslinking agent has been added, at a temperature of about 80°C to about 220°C for about 15 minutes to about 100 minutes. When the crosslinking reaction temperature is lower than 80°C, a sufficient surface crosslinking reaction may not occur, and when the crosslinking reaction temperature is higher than 220°C, an excessive surface crosslinking reaction may occur. Further, when the crosslinking reaction time is as short as less than 15 minutes, a sufficient crosslinking reaction may not occur, and when the crosslinking reaction time exceeds 100 minutes, the crosslinking density of the particle surface is excessively increased due to excessive surface crosslinking reaction, leading to deterioration in the physical properties. More specifically, the surface crosslinking reaction may be carried out by heating at a temperature of 120°C or higher, or 140°C or higher, and 200°C or lower, or 180°C or lower for 20 minutes or more, or 40 minutes or more, and 70 minutes or less, or 60 minutes or less.

A means for raising the temperature for the additional crosslinking reaction is not particularly limited. Heating may be performed by providing a heating medium or by directly providing a heat source. In this regard, the type of the heating medium applicable may be a hot fluid such as steam, hot air, hot oil, or the like. However, the present invention is not limited thereto. The temperature of the heating medium provided may be properly controlled, considering the means of the heating medium, the heating rate, and the target temperature. Meanwhile, as the heat source provided directly, an electric heater or a gas heater may be used, but the present invention is not limited to these examples.

Furthermore, provided is a hygiene product including the above-described superabsorbent polymer.

Hereinafter, the present invention will be described in more detail for better understanding. However, the following exemplary embodiments are only for illustrating the present invention, and the content of the present invention is not limited to the following exemplary embodiments.

**[Example: Preparation of superabsorbent polymer]**

**Example 1**

In a 3L glass container equipped with a stirrer, a nitrogen feeder, and a thermometer, 100 parts by weight of acrylic acid, 0.35 parts by weight of polyethylene glycol diacrylate (PEGDA, Mn= 575) as a first crosslinking agent, 0.008 parts by weight of bis(2,4,6-trimethylbenzoyl)-phenylphosphine oxide as a photoinitiator, 0.12 parts by weight of sodium persulfate (SPS) as a thermal initiator, 123.5 parts by weight of a sodium hydroxide solution with purity of 31.5%, 54.9 parts by weight of water, and 5 parts by weight of 2-(tert-butylamino)ethyl methacrylate (Sigma-Aldrich, product no. 444332) were added to prepare an aqueous solution of water-soluble unsaturated monomer while feeding nitrogen continuously.

The aqueous solution of water-soluble unsaturated monomer was transferred to a stainless steel container having a width of 250 mm, a length of 250 mm, and a height of 30 mm, and then subjected to ultraviolet irradiation (dose: 10 $mV/cm^2$) in a UV chamber at 80°C for 2 minutes and aged for 2 minutes to obtain a water-containing gel polymer.

The obtained water-containing gel polymer was pulverized into a size of 3 mm * 3 mm, and then the obtained gel-type polymer was spread on a stainless wire gauze having a hole size of 600 μm at a thickness of about 30 mm, and dried in a hot air oven at 120°C for 11 hours. The dried polymer thus obtained was pulverized using a pulverizer, and classified with a standard sieve according to ASTM to obtain a base polymer having a particle size of 150 μm to 850 μm.

Meanwhile, for surface crosslinking (additional crosslinking) of the base polymer, a surface crosslinking solution was prepared by mixing 5.4 parts by weight of water, 1.2 parts by weight of ethylene carbonate, and 0.2 parts by weight of propylene glycol, 0.2 parts by weight of a polycarboxylic acid surfactant, and 0.2 parts by weight of aluminum sulfate, based on 100 parts by weight of the base polymer. The surface crosslinking agent was sprayed onto 100 parts by weight of the base polymer using a paddle type mixer at 1000 rpm. Then, surface crosslinking was performed by heat treatment at the maximum temperature of 184 °C for 40 minutes to prepare a superabsorbent polymer of Example 1.

**Example 2**

A superabsorbent polymer was prepared in the same manner as in Example 1, except that 10 parts by weight of 2-(tert-butylamino)ethyl methacrylate was used and the surface crosslinking was performed at the maximum temperature of 178°C for 30 minutes in Example 1.

**Comparative Example 1**

A superabsorbent polymer was prepared in the same manner as in Example 1, except that 2-(tert-butylamino)ethyl methacrylate was not used in Example 1.

**Comparative Example 2**

2-(tert-butyl amino)ethyl methacrylate (18.5 g) and ethanol (30 mL) were added to a 2-neck round bottom flask capable of maintaining a nitrogen environment, followed by stirring for 10 minutes. Thereafter, azobisisobutyronitrile (AIBN (493 mg)) was added, followed by stirring at 80°C for 16 hours. The temperature of the reaction mixture was decreased to room temperature, and then a small amount of ethanol was added to dilute the solution. The diluted solution was slowly added dropwise to distilled water. The produced solid was filtered, washed with a small amount of distilled water, and dried to obtain 2-(tert-butyl amino)ethyl methacrylate polymer (Mw =32856, Mn =21055, Mp =49340, PD = 1.6).

A superabsorbent polymer mixture was prepared by mixing 5 parts by weight of 2-(tert-butylamino)ethyl methacrylate polymer with 100 parts by weight of the superabsorbent polymer prepared in the same manner as in Comparative Example 1.

**Comparative Example 3**

2-(tert-butyl amino)ethyl methacrylate (18.5 g) and ethanol (30 mL) were added to a 2-neck round bottom flask capable of maintaining a nitrogen environment, followed by stirring for 10 minutes. Thereafter, azobisisobutyronitrile (AIBN (493 mg)) was added, followed by stirring at 80°C for 16 hours. The temperature of the reaction mixture was decreased to room temperature, and then a small amount of ethanol was added to dilute the solution. The diluted solution was slowly added dropwise to distilled water. The produced solid was filtered, washed with a small amount of distilled water, and dried to obtain 2-(tert-butyl amino)ethyl methacrylate polymer (Mw =32856, Mn =21055, Mp =49340, PD = 1.6).

A superabsorbent polymer mixture was prepared by mixing 10 parts by weight of 2-(tert-butylamino)ethyl methacrylate polymer with 100 parts by weight of the superabsorbent polymer prepared in the same manner as in Comparative Example 1.

Mw, Mn, Mp and PD of 2-(tert-butyl amino)ethyl methacrylate polymers of Comparative Example 2 and Comparative Example 3 were measured by the following methods. The molecular weight distribution (PD=Mw/Mn) was determined by measuring Mw and Mn using gel permeation chromatography (GPC). The test was performed using a Waters E2695, 2414 RI instrument using a Polymer Laboratories's PLgel MIX-C/D 300 mm-length column. The test temperature was 40°C, N,N-dimethylformamide (DMF) was used as a solvent, and measurement was performed at a flow rate of 1 mL/min. Samples were prepared at a concentration of 5 mg/1 mL and then supplied in an amount of 100 μL. The values of Mw and Mn were derived using a calibration curve formed using polymethyl methacrylate (PMMA) standards. 9 kinds of polymethyl methacrylate standards having a molecular weight of 1,980 / 13,630 / 32,340 / 72,800 / 156,200 / 273,600 / 538,500 / 1,020,000 / 1,591,000 were used.

**[Experimental Example]**

(1) Evaluation of antibacterial property of polymerizable antibacterial monomer against E. *coli*

Antibacterial properties of the monomer 2-(tert-butylamino)ethyl methacrylate included in Examples and the following monomer 2-(dimethylamino)ethyl methacrylate were evaluated by the following methods.

The antibacterial property of 2-(tert-butylamino)ethyl methacrylate was shown in Experimental Examples 1-1 to 1-3, and the antibacterial property of 2-(dimethylamino)ethyl methacrylate was shown in Comparative Experimental Examples 1-1 to 1-4.

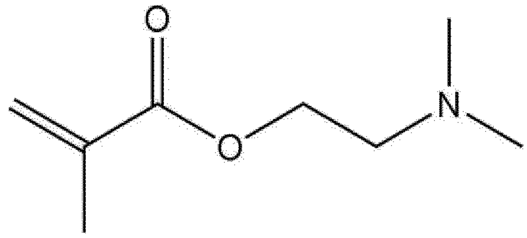

The monomer was put in a 50 ml conical tube according to the content of Table 1 below, and then 25 ml of a nutrient broth (Becton Dickinson) solution, to which 3000±300 CFU/ml of E. *coli* (ATCC 25922) standard strain was inoculated, was injected. After culturing for 18 hours in a shaking incubator (Vision Scientific, VS-37SIF) at 35°C, the culture solution was diluted to 1/5 concentration using 1X PBS (Gibco). The absorbance of the diluted solution was measured at a wavelength of 600 nm using a UV-Vis spectrophotometer. At this time, the absorbance measured after culturing the nutrient broth solution to which the bacteria was inoculated without the addition of the monomer was compared to the absorbance of the sample to which the monomer was added, and the degree of growth of the bacteria was examined. The bacterial growth-inhibitory rate was calculated according to the following Equation 1, and the results are shown in Table 1 below.

[Equation 1]

$$\text{Bacterial growth-inhibitory rate(\%)} = [(OD_{Reference} - OD_{Sample})/OD_{Reference}] * 100$$

in Equation, $OD_{Reference}$ represents absorbance of the culture medium of Comparative Experimental Example 1-1 without the antibacterial material, and $OD_{sample}$ represents absorbance of the culture medium with the antibacterial material.

[Table 1]

| | Kind of monomer | Content of monomer (g) | Optical density | Bacterial growth-inhibitory rate (%) |
|---|---|---|---|---|
| Experimental Example 1-1 | 2-(tert-butylamino)ethyl methacrylate | 0.03 | 0.185 | 5.61 |
| Experimental Example 1-2 | 2-(tert-butylamino)ethyl methacrylate | 0.05 | 0.070 | 64.29 |
| Experimental Example 1-3 | 2-(tert-butylamino)ethyl methacrylate | 0.07 | 0.009 | 95.41 |
| Comparative Experimental Example 1-1 | 2-(tert-butylamino)ethyl methacrylate | 0 | 0.196 | 0 |
| Comparative Experimental Example 1-2 | 2-(dimethylamino)ethyl methacrylate | 0.03 | 0.196 | 0.00 |
| Comparative Experimental Example 1-3 | 2-(dimethylamino)ethyl methacrylate | 0.05 | 0.189 | 3.67 |
| Comparative Experimental Example 1-4 | 2-(dimethylamino)ethyl methacrylate | 0.07 | 0.149 | 23.98 |

According to Table 1, when the polymerizable antibacterial monomer of the present invention was included, the bacterial growth-inhibitory rate was superior to that of the case where the different monomer 2-(dimethylamino)ethyl methacrylate of Comparative Experimental Example was used in the same amount, of which the terminal substituent was a tertiary amine group.

(2) Evaluation of antibacterial property of polymerizable antibacterial monomer against *Proteus mirabilis (P. mirabilis)*

To examine the antibacterial properties of the monomer 2-(tert-butylamino)ethyl methacrylate included in Examples and the monomer 2-(dimethylamino)ethyl methacrylate against *Proteus mirabilis,* the bacterial growth-inhibitory rate (%) against *Proteus mirabilis (P. mirabilis)* was calculated in the same manner as in the antibacterial property evaluation for *E. coli,* except that a nutrient broth solution, to which 3000±300 CFU/ml of *Proteus mirabilis* (P. mirabilis, ATCC 29906) standard strain was inoculated instead of *E. coli* (ATCC 25922), was used. The results are shown in Table 2 below.

In calculating the bacterial growth-inhibitory rate using Equation 1, $C_{Reference}$ represents CFU of bacteria after culturing with the monomer of Comparative Experimental Example 2-1 without the antibacterial material.

[Table 2]

| | Kind of monomer | Content of monomer (g) | Optical density | Bacterial growth-inhibitory rate (%) |
|---|---|---|---|---|
| Experimental Example 2-1 | 2-(tert-butylamino)ethyl methacrylate | 0.03 | 0.144 | 19.10 |
| Experimental Example 2-2 | 2-(tert-butylamino)ethyl methacrylate | 0.05 | 0.100 | 43.82 |
| Experimental Example 2-3 | 2-(tert-butylamino)ethyl methacrylate | 0.07 | 0.009 | 94.94 |
| Comparative Experimental Example 2-1 | 2-(tert-butylamino)ethyl methacrylate | 0 | 0.178 | 0 |
| Comparative Experimental Example 2-2 | 2-(dimethylamino)ethyl methacrylate | 0.03 | 0.172 | 3.37 |
| Comparative Experimental Example 2-3 | 2-(dimethylamino)ethyl methacrylate | 0.05 | 0.142 | 20.22 |
| Comparative Experimental Example 2-4 | 2-(dimethylamino)ethyl methacrylate | 0.07 | 0.057 | 67.98 |

According to Table 2, when the polymerizable antibacterial monomer of the present invention was included, the bacterial growth-inhibitory rate was superior to that of the case where the different monomer 2-(dimethylamino)ethyl methacrylate of Comparative Experimental Example was used in the same amount, of which the terminal substituent was a tertiary amine group.

(3) Evaluation of absorption properties of superabsorbent polymer

Centrifuge retention capacity (CRC) of the superabsorbent polymers of Examples and Comparative Examples was evaluated by the following method, and shown in Table 3.

The centrifuge retention capacity by absorption capacity under no load was measured for each of the superabsorbent polymers and base polymers of Examples and Comparative Examples in accordance with the European Disposables and Nonwovens Association (EDANA) WSP 241.3.

In detail, after uniformly introducing $W_0$(g) (about 0.2 g) of the superabsorbent polymer in a nonwoven fabric-made bag and sealing the same, it was immersed in physiological saline (0.9 wt% aqueous sodium chloride solution) at room temperature. After 30 minutes, the bag was drained using a centrifuge at 250 G for 3 minutes, and then the weight $W_2$(g) of the bag was measured. Further, after carrying out the same operation without using the polymer, the weight $W_1$(g) of the bag was measured. CRC (g/g) was calculated using each obtained weight according to the following Equation:

[Equation 3]

$$CRC\ (g/g) = \{[W_2(g) - W_1(g)]/W_0(g)\} - 1$$

in Equation 3,
$W_0$(g) is the initial weight (g) of the superabsorbent polymer,
$W_1$(g) is the weight of the bag, which was measured after the bag without the superabsorbent polymer was immersed in physiological saline for 30 minutes, and then drained using a centrifuge at 250 G for 3 minutes, and
$W_2$(g) is the weight of the bag including the superabsorbent polymer, which was measured after the superabsorbent polymer was immersed in physiological saline at room temperature for 30 minutes, and then drained using a centrifuge at 250 G for 3 minutes.

(4) Evaluation of antibacterial property of superabsorbent polymer against *Escherichia coli (E. coli)*

2 g of each superabsorbent polymer prepared in Examples and Comparative Examples was put in a 250 ml cell culture flask, and 50 ml of artificial urine, to which a test bacterium, *Escherichia coli (E. coli,* ATCC 25922) standard strain was inoculated at 3000±300 CFU/ml, was injected thereto. Thereafter, to allow the superabsorbent polymer to sufficiently absorb the artificial urine solution, they were mixed for about 1 minute. When the polymer sufficiently absorbed the solution, it exhibited a gel form, which was incubated in an incubator (JEIO TECH) at 35°C for 12 hours. To the sample, of which incubation was completed, 150 ml of 0.9 wt% NaCl solution was added, followed by shaking for about 1 minute. This dilution was spread on an agar medium plate. Thereafter, serial dilution was performed for colony counting, and in this procedure, 0.9 wt% NaCl solution was used. Antibacterial performance was determined by calculating a bacteriostatic reduction rate (%) against *E. coli* (ATCC 25922) according to the following Equation 1 after calculating the initial concentration of the bacteria (Co, CFU/ml) based on the dilution concentrations. The results are shown in Table 3 below.

[Equation 2]

$$\text{Bacteriostatic reduction rate}(\%) = (1 - C_{sample} / C_{Reference}) * 100$$

In calculating the bacteriostatic reduction rate using Equation 1, $C_{sample}$ represents CFU of bacteria after incubation of the superabsorbent polymer with the antibacterial material, and $C_{Reference}$ represents CFU of bacteria after incubation of the superabsorbent polymer of Comparative Example 1 without the antibacterial material.

(5) Evaluation of antibacterial property of superabsorbent polymer against *Proteus mirabilis (P. mirabilis)*

To examine antibacterial properties of the superabsorbent polymers prepared in Examples and Comparative Examples against *Proteus mirabilis (P. mirabilis),* the bacteriostatic reduction rate (%) against *Proteus mirabilis* was calculated in the same manner as in '(4) evaluating the antibacterial property of the superabsorbent polymer against *Escherichia coli (E.coli),* except that *Proteus mirabilis (P. mirabilis)* was used instead of *Escherichia coli (E.coli,* ATCC 25922) standard strain. The results are shown in Table 3 below.

(6) Evaluation of ammonia deodorization

Deodorization evaluation was performed on the superabsorbent polymers of Examples and Comparative Examples by the following method.

In detail, each of the superabsorbent polymers prepared in Examples and Comparative Examples was injected, at a concentration of 0.04 g/ml per 1 ml of solvent, into 50 ml of artificial urine, to which *Proteus Mirabilis* (ATCC 29906) was inoculated at 10,000 CFU/ml, followed by incubation in an incubator (JEIO TECH) at 35°C for 12 hours. After connecting an ammonia detection tube (Gastech, ammonia 3M) and a suitable pump (Gastech, GV-100) to the incubated vessel, 50 ml was extracted using a syringe needle. The color of the detection tube was changed by ammonia, and the scale was examined and compared. The composition of the artificial urine used at this time was prepared using a method described in J Wound Ostomy Continence Nurs. 2017; 44(1) 78-83. The results are shown in Table 3 below.

[Table 3]

| | Example 1 | Example 2 | Comparative Example 1 | Comparative Example 2 | Comparative Example 3 |
|---|---|---|---|---|---|
| CRC (g/g) of base polymer | 43.7 | 40.4 | 44.6 | 43.7 | 43.7 |
| CRC (g/g) of final superabsorbent polymer | 35.6 | 37.4 | 36.7 | 35.6 | 35.6 |
| Surface crosslinking time (min) | 40 | 30 | 40 | 40 | 40 |
| Surface crosslinking temperature (°C) | 184 | 178 | 184 | 184 | 184 |
| Bacteriostatic reduction rate (%) against *E.coli* | 47.05 | 95.49 | - | 0 | 4.05 |
| Bacteriostatic reduction rate (%) against *P. mirabilis* | 87.85 | 99.99 | - | 0 | 9.40 |
| Ammonia (ppm) | < 10 | < 10 | > 500 | > 500 | > 500 |

The results of Table 3 showed that the superabsorbent polymers of Examples 1 and 2 and Comparative Example 1 had the equivalent level of CRC by controlling the surface crosslinking time and temperature, whereas the bacteriostatic reduction rate against *Escherichia coli* and *Proteus mirabilis* was high, indicating antibacterial properties. In Comparative Examples 2 and 3, the superabsorbent polymer of Example 1 was used, and thus they had the same absorption properties as that of Example 1. However, it was confirmed that the antibacterial properties against *Escherichia coli* and *Proteus mirabilis* were significantly reduced only by simple mixing.

In addition, *Proteus mirabilis,* which is a microorganism having an enzyme such as urease, decomposes urea contained in artificial urine into the form of ammonia. Thus, as *Proteus mirabilis* proliferated, the amount of ammonia increased, and thus the odor of urine was greatly increased. As shown in Table 3, the generation of ammonia was greatly reduced in Examples, as compared to Comparative Examples, indicating the excellent deodorizing effect by antibacterial action.

## Claims

**1.** A superabsorbent polymer comprising a base polymer including a crosslinked polymer which is obtained by polymerizing an acrylic acid-based monomer including acidic groups of which at least part is neutralized, a polymerizable antibacterial monomer represented by the following Chemical Formula 1, and a first crosslinking agent,

wherein at least part of the base polymer is surface-treated by a second crosslinking agent:

[Chemical Formula 1]

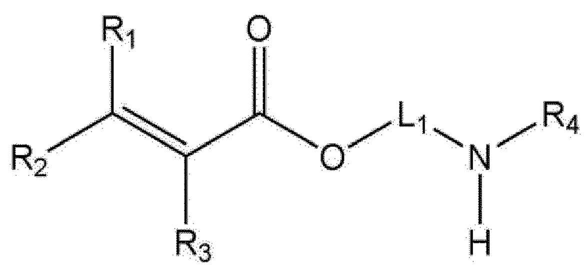

in Chemical Formula 1,
$R_1$ to $R_3$ are each independently hydrogen or methyl,
$R_4$ is hydrogen or a substituted or unsubstituted alkyl having 1 to 20 carbon atoms, and
$L_1$ is a substituted or unsubstituted alkylene having 1 to 10 carbon atoms.

**2.** The superabsorbent polymer of claim 1, wherein $R_1$ and $R_2$ are each hydrogen, and $R_3$ is methyl.

**3.** The superabsorbent polymer of claim 1, wherein $R_4$ is tert-butyl.

**4.** The superabsorbent polymer of claim 1, wherein $L_1$ is ethylene.

**5.** The superabsorbent polymer of claim 1, wherein the polymerizable antibacterial monomer is a compound represented by the following Chemical Formula 1-1:

[Chemical Formula 1-1]

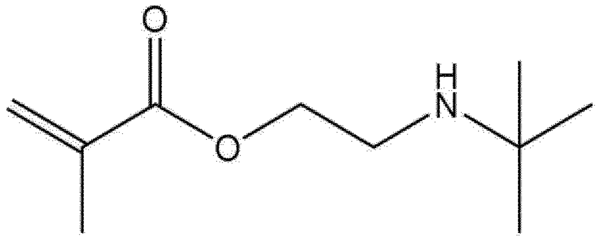

.

**6.** The superabsorbent polymer of claim 1, wherein the polymerizable antibacterial monomer is used in an amount of 5 parts by weight or more and 20 parts by weight or less with respect to 100 parts by weight of the acrylic acid-based monomer.

**7.** The superabsorbent polymer of claim 1, wherein the superabsorbent polymer exhibits an antibacterial property against the Gram-negative bacterium.

**8.** The superabsorbent polymer of claim 7, wherein the Gram-negative bacterium is *Escherichia coli* or *Proteus mirabilis.*

**9.** The superabsorbent polymer of claim 1, wherein the superabsorbent polymer exhibits a 30-min centrifuge retention capacity CRC of 20 g/g or more and 45 g/g or less for physiological saline which is 0.9 wt% aqueous sodium chloride solution, as measured according to the EDANA method WSP 241.3.

**10.** A method of preparing a superabsorbent polymer, the method comprising the steps of:

forming a water-containing gel polymer by performing a crosslinking polymerization of an acrylic acid-based monomer including acidic groups of which at least part is neutralized, and a polymerizable antibacterial monomer represented by the following Chemical Formula 1 in the presence of a first crosslinking agent and a polymerization initiator;
forming a base polymer including a crosslinked polymer by drying, pulverizing, and classifying the water-containing gel polymer; and
surface-crosslinking the base polymer by heat treatment in the presence of a second crosslinking agent:

[Chemical Formula 1]

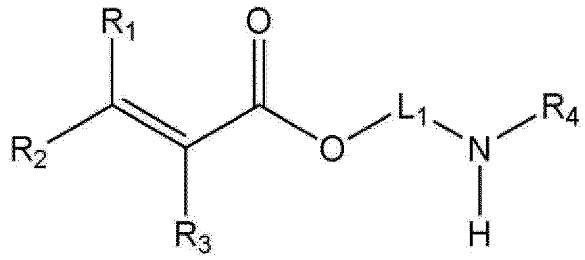

in Chemical Formula 1,
$R_1$ to $R_3$ are each independently hydrogen or methyl,
$R_4$ is hydrogen or a substituted or unsubstituted alkyl having 1 to 20 carbon atoms, and
$L_1$ is a substituted or unsubstituted alkylene having 1 to 10 carbon atoms.

**11.** The method of preparing a superabsorbent polymer of claim 10,
wherein the polymerizable antibacterial monomer is used in an amount of 5 parts by weight or more and 20 parts by weight or less with respect to 100 parts by weight of the acrylic acid-based monomer.

**12.** A hygiene product comprising the superabsorbent polymer of any one of claims 1 to 9.

**INTERNATIONAL SEARCH REPORT**

International application No.
**PCT/KR2022/012466**

**A. CLASSIFICATION OF SUBJECT MATTER**

**C08J 3/24**(2006.01)i; **C08F 220/34**(2006.01)i; **C08F 220/04**(2006.01)i; **A61L 15/60**(2006.01)i

According to International Patent Classification (IPC) or to both national classification and IPC

**B. FIELDS SEARCHED**

Minimum documentation searched (classification system followed by classification symbols)

C08J 3/24(2006.01); A01N 47/44(2006.01); A01N 61/00(2006.01); A01P 3/00(2006.01); A61L 9/01(2006.01); C08F 120/34(2006.01); C08F 2/06(2006.01); C08F 2/48(2006.01); C08F 220/34(2006.01); C08K 5/00(2006.01); C08K 5/20(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 항균(antibacterial), 고흡수성 수지(superabsorbent polymer, SAP), t-부틸 아미노 에틸 메타크릴레이트(t-butyl amino ethyl methacrylate)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2021-0058714 A (LG CHEM, LTD.) 24 May 2021 (2021-05-24)<br>See claims 1-11; and paragraphs [0033]-[0112]. | 1-12 |
| Y | JP 10-251350 A (HUELS AG) 22 September 1998 (1998-09-22)<br>See claim 1; and paragraphs [0051]-[0059]. | 1-12 |
| A | CN 101591408 A (SUN YAT-SEN UNIVERSITY) 02 December 2009 (2009-12-02)<br>See entire document. | 1-12 |
| A | KR 10-2020-0073750 A (LG CHEM, LTD.) 24 June 2020 (2020-06-24)<br>See entire document. | 1-12 |
| A | JP 6329229 B2 (NIPPON SHOKUBAI CO., LTD.) 23 May 2018 (2018-05-23)<br>See entire document. | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 December 2022** | **02 December 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2022/012466**

| Patent document cited in search report | | | Publication date (day/month/year) | Patent family member(s) | | | Publication date (day/month/year) |
|---|---|---|---|---|---|---|---|
| KR | 10-2021-0058714 | A | 24 May 2021 | CN | 114599696 | A | 07 June 2022 |
| | | | | EP | 4029375 | A1 | 20 July 2022 |
| | | | | US | 2022-0346379 | A1 | 03 November 2022 |
| | | | | WO | 2021-096230 | A1 | 20 May 2021 |
| JP | 10-251350 | A | 22 September 1998 | EP | 0862858 | A1 | 09 September 1998 |
| | | | | EP | 0862858 | B1 | 03 July 2002 |
| | | | | US | 5967714 | A | 19 October 1999 |
| | | | | US | 6203856 | B1 | 20 March 2001 |
| CN | 101591408 | A | 02 December 2009 | | None | | |
| KR | 10-2020-0073750 | A | 24 June 2020 | | None | | |
| JP | 6329229 | B2 | 23 May 2018 | JP | 2017-214346 | A | 07 December 2017 |
| | | | | JP | 2018-058813 | A | 12 April 2018 |
| | | | | JP | 2018-150310 | A | 27 September 2018 |
| | | | | JP | 7039121 | B2 | 22 March 2022 |
| | | | | WO | 2017-057571 | A1 | 06 April 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210120837
- KR 1020220103399


### Non-patent literature cited in the description


- **REINHOLD SCHWALM.** UV Coatings: Basics, Recent Developments and New Application. Elsevier, 2007, 115
- **ODIAN.** Principle of Polymerization. Wiley, 1981, 203
- *J Wound Ostomy Continence Nurs.,* 2017, vol. 44 (1), 78-83